# EUROPEAN PATENT SPECIFICATION

(11) **EP 3 522 177 B1**
(45) Date of publication and mention of the grant of the patent: **09.09.2020**
(21) Application number: 19154719.9
(22) Date of filing: 31.01.2019
(51) Int. Cl.: G21G 4/02, G21B 1/00, A61N 5/10, H05H 3/06

(54) **APPARATUS FOR INTRAOPERATIVE RADIOTHERAPY**
VORRICHTUNG FÜR INTRAOPERATIVE STRAHLENTHERAPIE
APPAREIL DE RADIOTHÉRAPIE INTRAOPÉRATIVE

(30) Priority: 02.02.2018 IT 201800002327
(43) Date of publication of application: 07.08.2019
(73) Proprietor: Martellini, Maurizio, 20142 Milano (IT); Gherardi, Giuseppe, 40126 Bologna (IT)
(72) Inventor: Martellini, Maurizio, 20142 Milano (IT); Gherardi, Giuseppe, 40126 Bologna (IT)
(74) Representative: Modiano, Micaela Nadia

(56) References cited:
- WO-A1-2017/164408
- US-A1- 2006 093 088
- US-A1- 2007 084 474
- US-A1- 2010 243 874
- US-A1- 2013 056 643

## Description

The present invention relates to an apparatus for intraoperative radiotherapy. Radiotherapy systems are disclosed in documents WO2017/164408 A1 and US2007/0084474 A1, for instance.

Intraoperative radiotherapy (IORT) is a treatment technique, in particular for treating tumors, which associates radiotherapy with surgery. This technique entails the administration of a dose of radiation directly on the tumor in order to eliminate the tissue that cannot be attacked surgically or, after having removed the neoplastic tissue, directly on the tumor bed.

One of the advantages of this technique is being able to apply the radiation directly on the affected tissue after it has been surgically exposed, thus sparing the healthy structures.

If needed, it is also possible to temporarily move the critical surrounding organs and shield the organs underneath during the administration of the radiotherapy.

The apparatuses nowadays commonly used to carry out this therapy comprise a source of radiation which emits, through an emission mouth, a beam of high-energy electrons and/or X-rays.

The radiation source is positioned so as to direct the beam of radiation directly on the tissue to be treated by way of an adjustable supporting structure which generally has an arm that can be rotated about the operating table.

Such conventional apparatuses, although very useful and practical, have a number of drawbacks.

First of all, in practice, the radiation emitted by these conventional apparatuses is absorbed unevenly by the tissues, in that the charged particles (such as electrons) deposit most of the total dose in a limited cross-section of the tissue (the region where absorption is concentrated is known as the Bragg peak).

Furthermore, the depth that can be reached in the irradiated tissue by the electrons emitted by conventional apparatuses is limited to around 3 cm.

X-ray radiation, on the other hand, passes through the entire body, irradiating the healthy tissues as well.

Another problem that is found in using conventional apparatuses is the exposure time necessary to reach the optimal dose for the therapy: this time, in some cases, can reach or exceed an hour and therefore, since it is impossible to keep the tissues immobile for such a long time, the exposure ends up being incorrect and uneven.

Furthermore, it would be desirable to have an apparatus available that provides a radiation flux with different physical characteristics from the flux provided by the known art, so as to be able to develop therapeutic protocols that are different and more efficacious.

The aim of the present invention consists in providing an apparatus for intraoperative radiotherapy (IORT) that solves the above technical problem, eliminates the drawbacks and overcomes the limitations of the known art, by making it possible to have available a different radiation flux which can be, at least in some respects, more efficacious with respect to the known art.

Within this aim, an object of the present invention is to provide an apparatus for intraoperative radiotherapy that provides a dose that is absorbed more evenly by the irradiated tissue.

Another object of the invention consists in providing an apparatus for intraoperative radiotherapy that provides a radiation that can reach a greater depth, and if possible controllable, in the irradiated tissue.

Another object of the invention consists in providing an apparatus for intraoperative radiotherapy that is capable of providing a same equivalent dose in a shorter time when compared to the known art.

Another object of the invention consists in providing an apparatus for intraoperative radiotherapy that can enable, at least in some situations, a more efficacious treatment.

This aim and these and other objects which will become better apparent hereinafter are achieved by an apparatus for intraoperative radiotherapy (IORT), which comprises a radiation generator which comprises an emission window, characterized in that said radiation generator is configured to emit, from said emission window, a mixed flow of neutrons and gamma photons and in that it comprises a beam shaping element for the moderation, reflection and collimation of the flow of neutrons and gamma photons emitted from said emission window.

Further characteristics and advantages of the invention will become better apparent from the description of two preferred, but not exclusive, embodiments of an apparatus for intraoperative radiotherapy which are illustrated by way of non-limiting example with the aid of the accompanying drawings wherein:
Figure 1 is a perspective view of a first possible embodiment of the apparatus for intraoperative radiotherapy, according to the invention;
Figure 2 is a perspective view of a second possible embodiment of the apparatus, according to the invention;
Figure 3 is a perspective view of an embodiment of the generator and of the beam shaping element, according to the invention;
Figure 4 is a cross-sectional view of the generator and of the beam shaping element in Figure 3, in a coupling configuration;
Figure 5 is a perspective view of the generator in Figure 3.

With reference to the figures, the apparatus for intraoperative radiotherapy, generally designated by the reference numeral 10, comprises a radiation generator 20 which in turn comprises an emission window 21 from which a radiation flux is emitted.

According to the invention, such radiation generator 20 is configured to emit, from the emission window 21, a mixed flow of neutrons and gamma photons 99.

Preferably, such generator 20 is constituted by a neutron generator with cylindrical geometry based on the deuterium-deuterium nuclear fusion reaction.

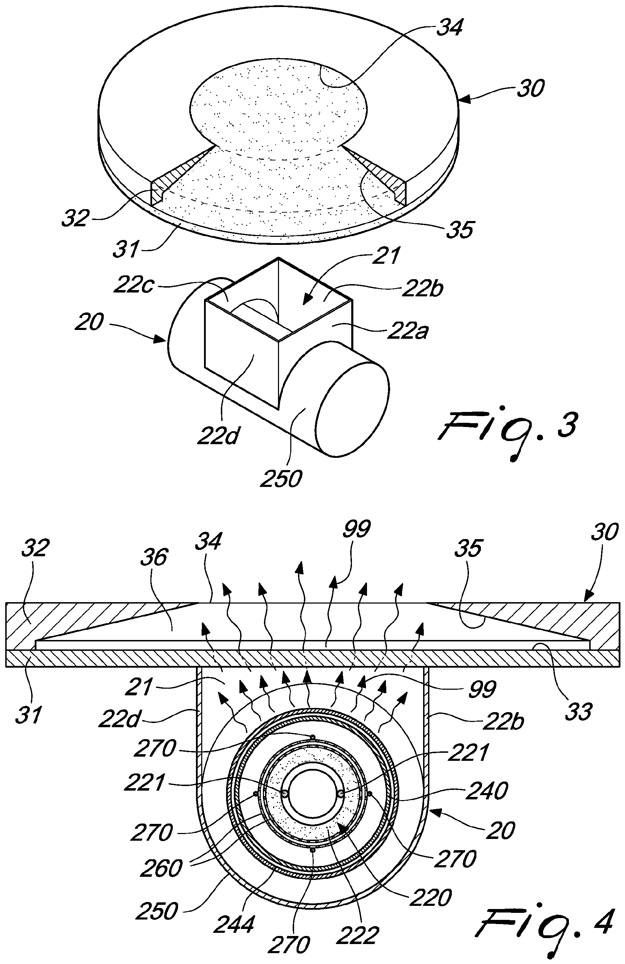

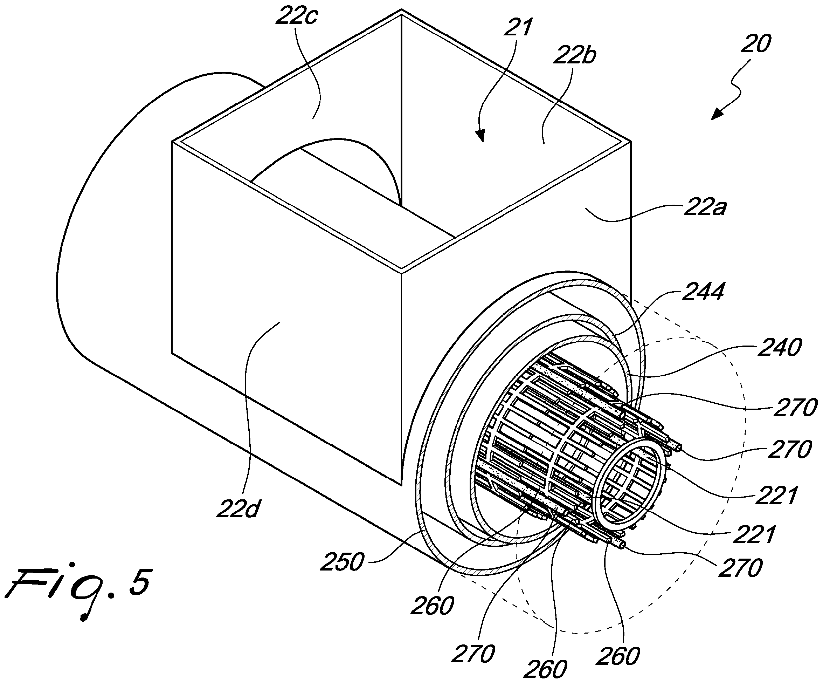

A preferred embodiment of the generator 20 is illustrated in Figures 3, 4 and 5. In this possible embodiment, the generator 20 comprises at least one source of deuterium ions 220 in which a deuterium gas is contained and which in turn comprises one or more radiofrequency antennas 221 for the creation of a plasma 222 of deuterium ions starting from such deuterium gas.

Such source of ions 220 is therefore configured to emit deuterium ions radially: in practice, the electromagnetic emission of the antennae 221 excites the deuterium gas, converting it to plasma 222 from which the deuterium ions are extracted, by virtue of the action of a grille of electrodes 260 positioned circumferentially to the source 220.

Positioned coaxial to the source of ions 220 is at least one cylindrical or conical target 240 which is configured so as to be struck by the ions that are emitted radially by the source 220 and, as a consequence, emitting radially neutrons and gamma photons 99.

The cylindrical or conical target 240 is constituted, for example, by a metal with high thermal conductivity covered by a thin layer with a high capacity for absorption of hydrogen, for example titanium, molybdenum or other material, with or without surface protection.

In this manner, during the operation, the absorbent layer is initially saturated by deuterium ions, originating from the source 220. The atoms of the absorbed deuterium then interact with other deuterium ions originating from the source 220 and generate the nuclear reactions that result in the emission of neutrons.

The cylindrical or conical target 240 can conveniently be surrounded by a containment wall 244, such as for example a quartz wall.

Naturally, the generator 20 also comprises all the further elements which, according to the known art, are necessary for the stability and the operation of this type of generator, such as for example magnets 270 for the confinement of the plasma.

The generator 20 further comprises a containment shell 250 which encloses the source of ions 222, the cylindrical or conical target 240 and the other elements.

As shown in Figures 3, 4 and 5, the containment shell 250 is provided with the aforementioned emission window 21 for the outward passage of at least some of the neutrons and gamma photons emitted by the cylindrical or conical target 240.

Preferably, the emission window 221 is quadrangular in shape, even more preferably square, and is surrounded by walls 22a, 22b, 22c, 22d that are mutually perpendicular and which extend outward from the containment shell 250.

In another possible embodiment, not shown, the position of the cylindrical or conical target 240 and of the source of ions 220 are inverted, with the cylindrical or conical target 240 contained inside the cylindrical source 220.

In yet another possible embodiment, not shown, there are a plurality of sources of ions and of cylindrical targets, in coaxial positions and alternately one inside the other.

In the preferred embodiment, the generator 20 therefore is substantially cylindrical and has a diameter comprised between 50 mm and 150 mm (even more preferably 100 mm) and a length comprised between 150 mm and 250 mm (even more preferably 200 mm).

Also in this embodiment, the emission window 21 has a width and a length comprised between 50mm and 150mm (even more preferably both are equal to 100 mm).

In more detail, the preferred generator 20 is configured so as to emit, from the emission window 21, between 10^9 and 10^12 neutrons per second, together with gamma photons.

According to the invention, the apparatus 10 also comprises a beam shaping element 30 for the moderation, reflection and collimation of the stream of neutrons and gamma photons 99 emitted from the emission window 21.

A possible embodiment of the beam shaping element is illustrated in Figures 3 and 4 and is substantially cylindrical with a diameter comprised preferably between 350 mm and 450 mm and a height preferably comprised between 50mm and 100 mm.

In more detail, in the preferred and illustrated embodiment, the beam shaping element 30 comprises a moderation layer 31 for the absorption and/or the slowing of at least some of the neutrons emitted by the generator 20.

Such moderation layer 31 is constituted by a layer of material that is capable of absorbing and/or slowing the neutrons with a speed that exceeds the speed predetermined in the design phase.

For example, in the preferred embodiments, the apparatus 10 is configured to emit thermal and/or epithermal neutrons and therefore the layer of moderation 31 is constituted by a material with high aptitude to convert the fast component of the neutron flux to thermal and epithermal neutrons, such as for example aluminum or aluminum fluoride or magnesium fluoride.

The beam shaping element 30 also comprises a collimation portion 32, adjacent to the moderation layer 31, the function of which is to collimate the flow of neutrons and gamma photons 99 in output from the moderation layer, imposing a preset geometry and spatial uniformity on them.

In even more detail, the collimation portion 32 comprises a larger inlet opening 33 which is adjacent to the moderation layer 31 and a smaller exit opening 34 which is coaxial and opposite with respect to such larger opening 33.

The larger opening 33 and the smaller opening 34 of the collimation portion 32 are connected by an internal wall 35 which defines a substantially frustum-shaped cavity 36 and is made of a material that is substantially impenetrable by neutrons.

Preferably, such internal wall 35 is constructed so as to reflect substantially all the neutrons, for example by being constituted by bismuth or by another material that reflects neutrons.

In other words, as can be seen in Figure 3, the beam shaping element 30 is shaped like a disk constituted by two superimposed disks: a first, solid disk which constitutes the moderation layer 31 and a second disk passed through by a frustum-shaped cavity 36 which constitutes the collimation portion 32.

Advantageously, the beam shaping element 30 is contained in a disk-shaped enclosure, free from corners and grooves and polished so as to be easy to clean and sterilize.

The geometry and the composition of the beam shaping element 30 (i.e. the thickness and the composition of the moderation layer 31 and the shape of the collimation portion 32) can be given, in the design phase, as a function of the intensity and of the composition of the radiation that it is desired to obtain, which, in turn, can be determined as a function of the equivalent dose required by the treatment for which the apparatus 10 is intended.

For example, the geometry and the composition of the beam shaping element 30 can be given using software simulations carried out using the Monte Carlo N-Particle Transport Code (MCNP).

In the preferred embodiment, the generator 20 and the beam shaping element 30 are configured so that a body exposed to the flow of neutrons and gamma photons 99 in output from the beam shaping element 30 absorbs an equivalent dose comprised between 10 Sv and 25 Sv in a time comprised between 5 minutes and 1 hour.

The apparatus 10 also comprises, preferably, a movable and adjustable supporting structure 400, which comprises at least one rotating arm 401 which can be oriented about an operating table b.

Such rotating arm 401 supports at least the generator 20 so as to make it selectively positionable at any anatomical portion of the body of a patient P lying on such operating table b.

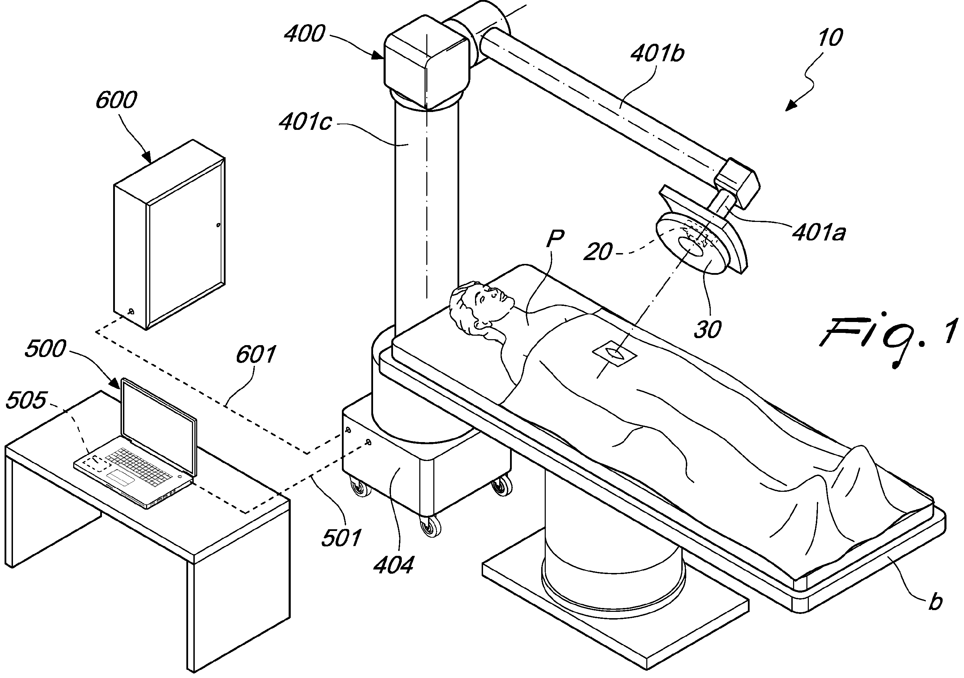

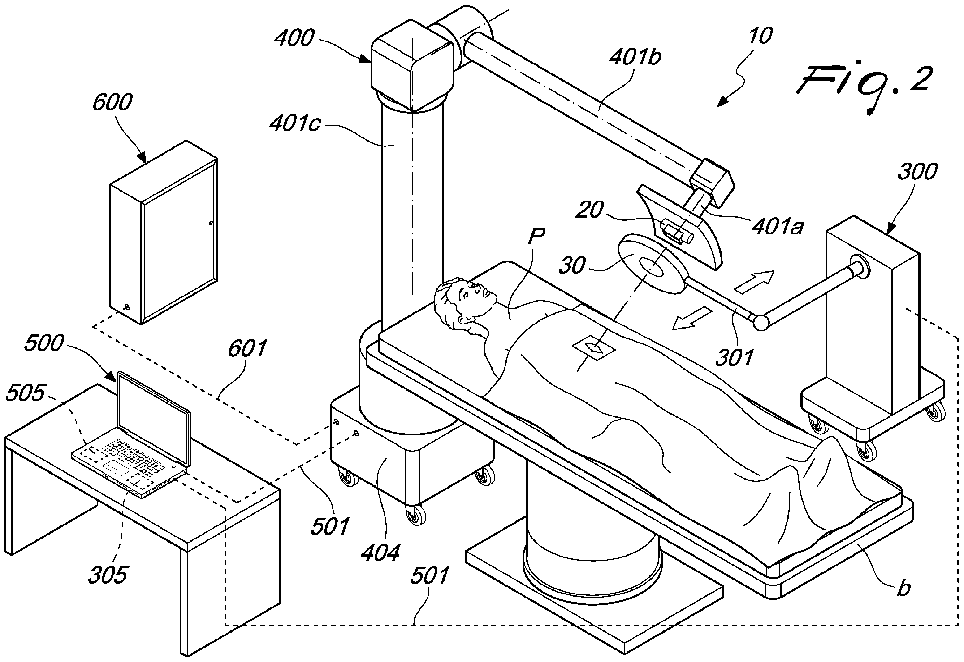

Figures 1 and 2 schematically illustrate a non-limiting example of the supporting structure that comprises a plurality of arms 401a, 401b, 401c that can mutually rotate with respect to each other and a movable base 404 for the translation of the supporting structure 400 within the operating theater.

In other embodiments, not shown, the supporting structure 400 is constituted by any structure in the known art, chosen from among those used for intraoperative radiotherapy and provided with at least one rotating arm, such as for example a structure which comprises an arm shaped like an upturned L which rotates about the horizontal axis and is connected to a fixed wall.

Figure 1 shows a first possible embodiment of the apparatus 10 overall. In this first embodiment, the beam shaping element 30 is fixed to the generator 20 so as to cover the emission window 21 (i.e. so as to cover its opening and so block all the radiation 99 in output from the emission window 21, as shown in Figure 4).

The generator 20, together with the beam shaping element 30, is in turn fixed to a rotatable arm 401 of the supporting structure 400.

By virtue of the reduced dimensions of the generator 20, the supporting structure 400 can have limited dimensions: for example the preferred supporting structure has a height less than 220 cm, a width less than 100 cm, a length less than 200 cm and a weight less than 500 kg.

Figure 2 shows a second possible embodiment of the apparatus 10 overall. In this second embodiment the beam shaping element 30 is coupled to a movable support 300, so as to be able to move independently of the generator 20.

Such movable support 300 is configurable at least in a coupling configuration, in which the beam shaping element 30 is arranged adjacent to the generator 20 at the emission window 21.

In practice, in this second embodiment, by moving the movable support 300 it is possible to position the beam shaping element 30 in front of the emission window 21 of the generator 20, interposing it between the generator 20 and the patient P.

The positioning of the beam shaping element 30 (i.e. the position, the distance and the orientation with respect to the generator 20) in some embodiments is executed manually by an operator, while in others it is executed automatically or semi-automatically by way of a remote control system 305.

Preferably, the movable support 300 comprises an adjustable arm 301 which supports the beam shaping element 30.

In the example shown, the movable support 300 is controlled by an electronic remote control system 305 which is connected to such movable support 300 by way of data transfer means 501 (such as for example data cables or a wireless system). Furthermore the movable support 300 is preferably provided with an optical positioning system (not shown), which is connected to the electronic remote control system 305, for controlling the positioning of the beam shaping element 30 with respect to the generator 20.

The optical positioning system is in practice an electronic system that comprises optical sensors that are configured to detect the position, distance and orientation of the beam shaping element 30 with respect to the generator 20 and to send feedback to the electronic remote control system 305, thus enabling the precise and automatic positioning.

In both of the two embodiments illustrated, the apparatus 10 also comprises an electronic control unit 505, which is connected by virtue of data transfer means 501 to the movable supporting structure 400, for controlling at least the orientation of the rotating arm 401a, 401b, 401c.

In the second embodiment, the electronic control unit 505 and the electronic remote control system 305 can be integrated in a same information technology system 500.

The apparatus 10 is further connected, preferably by way of flexible cables 601, to an electronic controller 600 for supplying power to the generator 20 and/or to the movable supporting structure 400.

In the preferred embodiment, the electronic controller 60 supplies electric power comprised between 50 kW and 300 kW according to the generator 20.

Optionally, the apparatus 10 also comprises a computerized system for locating the position of the elements for controlling and correcting the position of the generator 20 and/or of the beam shaping element 30 with respect to one or more organs of the patient P. A possible embodiment of the computerized localization system is represented by the system known as Calypso® 4D Localization System™.

Such computerized localization system can also be fully or partially integrated in the information technology system 500.

Advantageously, the information technology system 500 and the electronic controller 600 can be positioned outside the operating theater, allowing the control of the apparatus 10 by an operator located in a different room from the operating theater.

Operation of the apparatus for intraoperative radiotherapy is briefly described below.

Inside the operating theater, the generator 20 and the beam shaping element 30 are positioned, by way of the supporting structure 400 (and optionally by way of the movable support 300 which supports the beam shaping element 30) close to the patient P so as to direct the flow of neutrons and gamma photons 99 toward the tissue to be treated.

At this point the radiation generator 20 is activated and emits a mixed flow of neutrons and gamma photons 99 from the emission window 21.

The flow of neutrons and gamma photons 99 then passes through the beam shaping element 30 which moderates it, absorbing some of the neutrons, and imposes geometry and spatial uniformity adapted to the therapy on it.

The moderated flow then strikes the tissue to be treated. By prolonging exposure for a suitable period of time, the equivalent dose required by the therapy is therefore delivered.

It is to be noted that in one of the preferred embodiments described herein, with a generator 20 configured to emit 10^12 neutrons per second, an equivalent dose of 10 Sv is delivered in less than 5 minutes; while with a generator 20 configured to emit 10^10 neutrons per second, an equivalent dose of 10 Sv is delivered in about an hour.

It is also important to note that a flow of neutrons and gamma photons like that emitted by the apparatus 10, according to the present invention, deposits the radiation dose in the irradiated tissue spatially more evenly than a stream of charged particles (such as electrons).

Furthermore, the apparatus 10, according to the invention, makes it possible to obtain a flow of thermal neutrons that is adapted to carry out the radiotherapy technique known as "boron neutron capture therapy" (BNCT), which clinical trials have shown to be more efficacious in the treatment of some kinds of tumor, since it can count on a higher relative biological effectiveness (RBE).

In addition, the flow of neutrons and gamma photons 99 can be modulated, by way of the configuration of the generator 20 and of the beam shaping element 30, so as to select the depth of absorption of the radiation dose in the tissue, up to a depth of approximately 6 cm.

In practice it has been found that the apparatus for intraoperative radiotherapy, according to the present invention, achieves the intended aim and objects in that it makes it possible to have available a different stream of radiation, which is, at least in some respects, more efficacious with respect to the known art.

Another advantage of the apparatus for intraoperative radiotherapy, according to the invention, consists in that it delivers a dose that is absorbed more evenly by the irradiated tissue.

Another advantage of the apparatus for intraoperative radiotherapy, according to the invention, consists in that it provides a radiation that can reach a greater depth and is controllable in the irradiated tissue.

Another advantage of the apparatus for intraoperative radiotherapy, according to the invention, consists in that it provides a same equivalent dose in a shorter time when compared to the known art.

Another advantage of the apparatus for intraoperative radiotherapy, according to the invention, consists in that it makes a more efficacious treatment possible, at least in some situations.

The apparatus for intraoperative radiotherapy thus conceived is susceptible of numerous modifications and variations all of which are within the scope of the appended claims.

Moreover, all the details may be substituted by other, technically equivalent elements.

In practice the materials employed, provided they are compatible with the specific use, and the contingent dimensions and shapes, may be any according to requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus (10) for intraoperative radiotherapy (IORT), which comprises a radiation generator (20) which comprises an emission window (21), said radiation generator (20) being configured to emit, from said emission window (21), a mixed flow of neutrons and gamma photons (99), **characterized in that** it comprises a beam shaping element (30) for the moderation, reflection and collimation of the stream of neutrons and gamma photons (99) emitted from said emission window (21).

2. The apparatus according to claim 1, **characterized in that** said generator (20) is a neutron generator with cylindrical geometry based on the deuterium-deuterium nuclear fusion reaction.

3. The apparatus (10) according to claim 2, **characterized in that** said generator (20) comprises:
- at least one source of deuterium ions (220), which is adapted to contain a deuterium gas and comprises one or more radiofrequency antennas (221) for the creation of a plasma (222) of deuterium ions starting from said deuterium gas, said source of ions (220) being configured to emit deuterium ions radially;
- at least one cylindrical or conical target (240) for the generation of neutrons, which is coaxial to said source of ions (220) and configured to be struck by the deuterium ions emitted by said source of ions (220) and consequently to radially emit neutrons and gamma photons (99);
- a containment shell (250), which encloses said source of ions (220) and said cylindrical or conical target (240), said containment shell (250) being provided with said emission window (21) for the outward passage of at least some of the neutrons and gamma photons emitted by said target (240).

4. The apparatus (10) according or to one or more of the preceding claims, **characterized in that** said beam shaping element (30) comprises:
- a moderation layer (31) for the absorption and/or slowing of at least some of the neutrons emitted by said generator (20);
- a collimation portion (32), which comprises a larger inlet opening (33) which is adjacent to said moderation layer and a smaller exit opening (34) which is coaxial and opposite with respect to said larger opening; said larger opening (33) and said smaller opening (34) being connected by an internal wall (35) which defines a substantially frustum-shaped cavity (36) and is made of a material that is substantially impenetrable by neutrons.

5. The apparatus (10) according to claim 4, **characterized in that** said internal wall (35) reflects neutrons, said internal wall (35) being constituted by bismuth.

6. The apparatus (10) according to one or more of the preceding claims, **characterized in that** said beam shaping element (30) is affixed to said generator (20) in such a manner as to cover said emission window (21).

7. The apparatus (10) according to one or more of the preceding claims, **characterized in that** said beam shaping element (30) is coupled to a movable support (300); said movable support (300) being configurable at least in a coupling configuration, in which said beam shaping element (30) is arranged adjacent to said generator (20) at said emission window (21).

8. The apparatus according to claim 7, **characterized in that** said movable support (300) is controlled by an electronic remote control system (305) which is connected to said movable support by virtue of data transfer means (501); said movable support (300) being provided with an optical positioning system, which is connected to said electronic remote control system (305), for controlling the positioning of said beam shaping element (30) with respect to said generator (20).

9. The apparatus (10) according to one or more of the preceding claims, **characterized in that** said generator (20) is substantially cylindrical, having a diameter comprised between 50 mm and 150 mm and a length comprised between 150 mm and 250 mm,
and **in that** said beam shaping element (30) is substantially cylindrical, having a diameter comprised between 350 mm and 450 mm and a height comprised between 50 mm and 100 mm,
said emission window (21) having a substantially quadrangular cross-section with a width and length comprised between 50 mm and 150 mm.

10. The apparatus (10) according to one or more of the preceding claims, **characterized in that** said generator (20) is configured to emit from said emission window (21) between 10^9 and 10^12 neutrons per second and said beam shaping element (30) is configured so that a body that is exposed to the flow of neutrons and gamma photons in output from said beam shaping element (30) absorbs an equivalent dose comprised between 10 Sv and 25 Sv in a time comprised between 5 minutes and 1 hour.

11. The apparatus (10) according to one or more of the preceding claims, **characterized in that** it comprises a movable and adjustable supporting structure (400) which comprises at least one rotating arm (401a, 401b, 401c) which can be oriented around an operating table (b), said rotating arm (401a, 401b, 401c) supporting at least said generator (20) so as to make it substantially positionable at any anatomical portion of the body of a patient (P) lying on said operating table (b).

12. The apparatus (10) according to claim 11, **characterized in that** it also comprises:
- an electronic control unit (505), which is connected by virtue of data transfer means (501) to said movable supporting structure (400), for controlling at least the orientation of said rotating arm (401a, 401b, 401c);
- a computerized system for locating the position of the elements for controlling and correcting the position of said generator (20) and/or of said beam shaping element (30) with respect to one or more organs of the patient (P).

## Patentansprüche

1. Eine Vorrichtung (10) für intraoperative Strahlentherapie, intraoperative radiotherapy - IORT -, die einen Strahlungsgenerator (20) umfasst, welcher ein Emissionsfenster (21) umfasst,
wobei der Strahlungsgenerator (20) konfiguriert ist, um durch das Emissionsfenster (21) einen gemischten Strom aus Neutronen und gamma-Photonen (99) auszusenden,
**dadurch gekennzeichnet, dass** er ein Strahlenformelement (30) für die Moderierung, Reflektion und Kollimation des Stroms von Neutronen und gamma-Photonen (99) umfasst, die durch das Emissionsfenster (21) ausgesendet werden.

2. Die Vorrichtung gemäß Anspruch 1, **dadurch gekennzeichnet, dass** der Generator (20) ein Neutronengenerator mit zylindrischer Geometrie ist, der auf der Deuterium-Deuterium-Kernfusionsreaktion basiert.

3. Die Vorrichtung (10) gemäß Anspruch 2, **dadurch gekennzeichnet, dass** der Generator (20) Folgendes umfasst:
- mindestens eine Quelle für Deuteriumionen (220), die ausgebildet ist, um ein Deuteriumgas zu enthalten, und eine oder mehrere Hochfrequenzantennen (221) für die Erzeugung eines Plasmas (222) von Deuteriumionen, ausgehend von dem Deuteriumgas, umfasst; wobei die Quelle der Ionen (220) konfiguriert ist, um Deuteriumionen radial auszusenden;
- mindestens ein zylindrisches oder kegelförmiges Ziel (240) für die Erzeugung von Neutronen, das koaxial mit der Quelle für Ionen (220) und konfiguriert ist, um von den Deuteriumionen getroffen zu werden, die von der Quelle für Ionen (220) ausgesendet werden, und folglich radial Neutronen und gamma-Photonen (99) auszusenden;
- ein Einschlussgehäuse (250), das die Quelle für Ionen (220) und das zylindrische oder kegelförmige Ziel (240) einschließt; wobei das Einschlussgehäuse (250) mit dem Emissionsfenster (21) für den Durchlass mindestens einiger der Neutronen und gamma-Photonen, die von dem Ziel (240) ausgesendet werden, nach außen ausgestattet ist.

4. Die Vorrichtung (10) gemäß einem oder mehreren der obigen Ansprüche, **dadurch gekennzeichnet, dass** das Strahlenformelement (30) Folgendes umfasst:
- eine Moderierungsschicht (31) für die Absorption und/oder Abbremsung mindestens einiger der Neutronen, die von dem Generator (20) ausgesendet werden;
- einen Kollimationsabschnitt (32), der eine größere Einlassöffnung (33) umfasst, die an die Moderierungsschicht angrenzt, und eine kleinere Austrittsöffnung (34), die koaxial mit der größeren Öffnung ist und ihr gegenüberliegt; wobei die größere Öffnung (33) und die kleinere Öffnung (34) durch eine Innenwand (35) verbunden sind, die einen im Wesentlichen kegelstumpfförmigen Hohlraum (36) bestimmt und aus einem Material besteht, das für Neutronen im Wesentlichen undurchlässig ist.

5. Die Vorrichtung (10) gemäß Anspruch 4, **dadurch gekennzeichnet, dass** die Innenwand (35) Neutronen reflektiert, wobei die Innenwand (35) aus Wismut besteht.

6. Die Vorrichtung (10) gemäß einem oder mehreren der obigen Ansprüche, **dadurch gekennzeichnet, dass** das Strahlenformelement (30) an dem Generator (20) so befestigt ist, dass es das Emissionsfenster (21) bedeckt.

7. Die Vorrichtung (10) gemäß einem oder mehreren der obigen Ansprüche, **dadurch gekennzeichnet, dass** das Strahlenformelement (30) mit einem beweglichen Träger (300) gekoppelt ist, wobei der bewegliche Träger (300) mindestens in einer Kopplungskonfiguration konfigurierbar ist, in welcher das Strahlenformelement (30) angrenzend an den Generator (20) an dem Emissionsfenster (21) angeordnet ist.

8. Die Vorrichtung gemäß Anspruch 7, **dadurch gekennzeichnet, dass** der bewegliche Träger (300) durch ein elektronisches Fernsteuerungssystem (305) gesteuert wird, welches mit dem beweglichen Träger über Datenübertragungsmittel (501) verbunden ist, wobei der bewegliche Träger (300) mit einem optischen Positioniersystem ausgestattet ist, das zum Steuern der Positionierung des Strahlenformelements (30) im Verhältnis zu dem Generator (20) mit dem elektronischen Fernsteuerungssystem (305) verbunden ist.

9. Die Vorrichtung (10) gemäß einem oder mehreren der obigen Ansprüche, **dadurch gekennzeichnet, dass** der Generator (20) im Wesentlichen zylindrisch ist, einen Durchmesser zwischen 50mm und 150 mm und eine Länge zwischen 150 mm und 250 mm hat,
und dadurch, dass das Strahlenformelement (30) im Wesentlichen zylindrisch ist und einen Durchmesser zwischen 350mm und 450 mm und eine Höhe zwischen 50 mm und 100 mm hat, wobei das Emissionsfenster (21) einen im Wesentlichen viereckigen Querschnitt mit einer Breite und Länge zwischen 50 mm und 150 mm hat.

10. Die Vorrichtung (10) gemäß einem oder mehreren der obigen Ansprüche, **dadurch gekennzeichnet, dass** der Generator (20) konfiguriert ist, um durch das Emissionsfenster (21) zwischen 10^9 und 10^12 Neutronen pro Sekunde auszusenden, und das Strahlenformelement (30) so konfiguriert ist, dass ein Körper, der dem Strom von Neutronen und gamma-Photonen ausgesetzt ist, der von dem Strahlenformelement (30) kommt, in einer Zeit zwischen 5 Minuten und 1 Stunde eine Äquivalentdosis aufnimmt, die zwischen 10 Sv und 25 Sv beträgt.

11. Die Vorrichtung (10) gemäß einem oder mehreren der obigen Ansprüche, **dadurch gekennzeichnet, dass** sie eine bewegliche und verstellbare tragende Struktur (400) umfasst, die mindestens einen Rotationsarm (401a, 401b, 401c) umfasst, der um einen Operationstisch (b) herum ausgerichtet werden kann, wobei der Rotationsarm (401a, 401b, 401c) mindestens den Generator (20) trägt, um ihn im Wesentlichen an jedem Körperteil eines Patienten (P) positionierbar zu machen, der auf dem Operationstisch (b) liegt.

12. Die Vorrichtung (10) gemäß Anspruch 11, **dadurch gekennzeichnet, dass** sie außerdem Folgendes umfasst:
- eine elektronische Steuereinheit (505), die über Datenübertragungsmittel (501) mit der beweglichen tragenden Struktur (400) verbunden ist, um mindestens die Ausrichtung des Rotationsarms (401a, 401b, 401c) zu steuern;
- ein computerisiertes System zur Lokalisierung der Position der Elemente, um die Position des Generators (20) und/oder des Strahlenformelements (30) im Verhältnis zu einem oder mehreren Organen des Patienten (P) zu steuern und zu korrigieren.

## Revendications

1. Appareil (10) de radiothérapie peropératoire (IORT), qui comporte un générateur de rayonnement (20) qui comporte une fenêtre d'émission (21),
ledit générateur de rayonnement (20) étant configuré pour émettre, à partir de ladite fenêtre d'émission (21), un flux mixte de neutrons et de photons gamma (99),
**caractérisé en ce qu'**il comporte un élément de mise en forme de faisceau (30) pour la modération, la réflexion et la collimation du flot de neutrons et de photons gamma (99) émis à partir de ladite fenêtre d'émission (21).

2. Appareil selon la revendication 1, **caractérisé en ce que** ledit générateur (20) est un générateur de neutrons à géométrie cylindrique basé sur la réaction de fusion nucléaire deutérium-deutérium.

3. Appareil (10) selon la revendication 2, **caractérisé en ce que** ledit générateur (20) comporte :
- au moins une source d'ions deutérium (220), qui est adaptée pour contenir un gaz deutérium et comporte une ou plusieurs antennes radiofréquence (221) pour la création d'un plasma (222) d'ions deutérium en partant dudit gaz deutérium, ladite source d'ions (220) étant configurée pour émettre des ions deutérium radialement,
- au moins une cible cylindrique ou conique (240) pour la génération de neutrons, qui est coaxiale à ladite source d'ions (220) et configurée pour être frappée par les ions deutérium émis par ladite source d'ions (220) et par conséquent pour émettre radialement des neutrons et des photons gamma (99),
- une enveloppe de confinement (250), qui entoure ladite source d'ions (220) et ladite cible cylindre ou conique (240), ladite enveloppe de confinement (250) étant pourvue de ladite fenêtre d'émission (21) pour le passage vers l'extérieur d'au moins une partie des neutrons et des photons gamma émis par ladite cible (240) .

4. Appareil (10) selon une ou plusieurs des revendications précédentes, **caractérisé en ce que** ledit élément de mise en forme de faisceau (30) comporte :
- une couche de modération (31) pour l'absorption et/ou le ralentissement d'au moins une partie des neutrons émis par ledit générateur (20),
- une partie de collimation (32), qui comporte un orifice d'entrée plus grand (33) qui est adjacent à ladite couche de modération et un orifice de sortie (34) plus petit qui est coaxial et opposé par rapport audit orifice plus grand, ledit orifice plus grand (33) et ledit orifice plus petit (34) étant reliés par une paroi interne (35) qui définit une cavité de forme sensiblement tronconique (36) et est constituée d'un matériau qui est sensiblement impénétrable aux neutrons.

5. Appareil (10) selon la revendication 4, **caractérisé en ce que** ladite paroi interne (35) réfléchit les neutrons, ladite paroi interne (35) étant constituée de bismuth.

6. Appareil (10) selon une ou plusieurs des revendications précédentes, **caractérisé en ce que** ledit élément de mise en forme de faisceau (30) est fixé audit générateur (20) de telle manière à recouvrir ladite fenêtre d'émission (21).

7. Appareil (10) selon une ou plusieurs des revendications précédentes, **caractérisé en ce que** ledit élément de mise en forme de faisceau (30) est couplé à un support mobile (300), ledit support mobile (300) étant configurable au moins dans une configuration de couplage, dans laquelle ledit élément de mise en forme de faisceau (30) est agencé au voisinage dudit générateur (20) sur ladite fenêtre d'émission (21).

8. Appareil selon la revendication 7, **caractérisé en ce que** ledit support mobile (300) est commandé par un système de commande à distance électronique (305) qui est relié audit support mobile par des moyens de transfert de données (501), ledit support mobile (300) étant pourvu d'un système de positionnement optique, qui est relié audit système de commande à distance électronique (305), pour commander le positionnement dudit élément de mise en forme de faisceau (30) par rapport audit générateur (20).

9. Appareil (10) selon une ou plusieurs des revendications précédentes, **caractérisé en ce que** ledit générateur (20) est sensiblement cylindrique, ayant un diamètre compris entre 50 mm et 150 mm et une longueur comprise entre 150 mm et 250 mm,
et **en ce que** ledit élément de mise en forme de faisceau (30) est sensiblement cylindrique, ayant un diamètre compris entre 350 mm et 450 mm et une hauteur comprise entre 50 mm et 100 mm,
ladite fenêtre d'émission (21) ayant une section transversale sensiblement quadrangulaire avec une largeur et une longueur comprises entre 50 mm et 150 mm.

10. Appareil (10) selon une ou plusieurs des revendications précédentes, **caractérisé en ce que** ledit générateur (20) est configuré pour émettre à partir de ladite fenêtre d'émission (21) entre 10^9 et 10^12 neutrons par seconde et ledit élément de mise en forme de faisceau (30) est configuré de sorte qu'un corps qui est exposé au flux de neutrons et de photons gamma en sortie dudit élément de mise en forme de faisceau (30) absorbe une dose équivalente comprise entre 10 Sv et 25 Sv dans un temps compris entre 5 minutes et 1 heure.

11. Appareil (10) selon une ou plusieurs des revendications précédentes, **caractérisé en ce qu'**il comporte une structure de support mobile et réglable (400) qui comporte au moins un bras rotatif (401a, 401b, 401c) qui peut être orienté autour d'une table d'opération (b), ledit bras rotatif (401a, 401b, 401c) supportant au moins ledit générateur (20) de manière à pouvoir pratiquement le positionner sur toute partie anatomique du corps d'un patient (P) reposant sur ladite table d'opération (b).

12. Appareil (10) selon la revendication 11, **caractérisé en ce qu'**il comporte également :
- une unité de commande électronique (505), qui est reliée par des moyens de transfert de données (501) à ladite structure de support mobile (400), pour commander au moins l'orientation dudit bras rotatif (401a, 401b, 401c),
- un système informatisé pour localiser la position des éléments pour commander et corriger la position dudit générateur (20) et/ou dudit élément de mise en forme de faisceau (30) par rapport à un ou plusieurs organes du patient (P).
